# EUROPÄISCHE PATENTSCHRIFT

(11) **EP 1 356 080 B1**
(45) Veröffentlichungstag und Bekanntmachung des Hinweises auf die Patenterteilung: **03.05.2006**
(21) Anmeldenummer: 01913568.0
(22) Anmeldetag: 01.02.2001
(51) Int. Cl.: C12Q 1/04, G01N 33/569, C07K 14/01

(54) **NACHWEIS UND IDENTIFIKATION VON BAKTERIENGRUPPEN**
DETECTION AND IDENTIFICATION OF GROUPS OF BACTERIA
DETECTION ET IDENTIFICATION DE GROUPES DE BACTERIES

(43) Veröffentlichungstag der Anmeldung: 29.10.2003
(73) Patentinhaber: Profos AG, 93053 Regensburg (DE)
(72) Erfinder: MILLER, Stefan, 93059 Regensburg (DE)
(74) Vertreter: Dehmel, Albrecht
(86) Internationale Anmeldenummer: PCT/DE2001/000431
(87) Internationale Veröffentlichungsnummer: WO 2002/061117

(56) Entgegenhaltungen:
- WO-A-01/09370
- WO-A-93/17129
- BENNETT A R ET AL: "The use of bacteriophage-based systems for the separation and concentration of Salmonella." JOURNAL OF APPLIED MICROBIOLOGY, Bd. 83, Nr. 2, 1997, Seiten 259-265, XP001051057 ISSN: 1364-5072
- DATABASE EMBL [Online] ID: BT2GP12 AC: X56555, 28. Oktober 1992 (1992-10-28) MESYANZHINOV, V. V.: "Bacteriophage T2 gene for gp12" XP002187843
- SELIVANOV N A ET AL: "NUCLEOTIDE AND DEDUCED AMINO ACID SEQUENCE OF BACTERIOPHAGE T4 GENE 12" NUCLEIC ACIDS RESEARCH, Bd. 16, Nr. 5 PART B, 1988, Seite 2334 XP001052994 ISSN: 0305-1048 -& DATABASE EMBL [Online] ID: Myt4G12 AC: X06792, 2. April 1988 (1988-04-02) MESYANZHINOV, V.V.: "Enterobacteria phage T4" XP002187844

### SEQUENZPROTOKOLL

<110> PROFOS GmbH
<120> Nachweis und Identifikation von Bakteriengruppen
<130> PRO-002 PCT
<140> XX
   <141> 2001-02-01
<160> 4
<170> Patent In Ver. 2.1
<210> 1
   <211> 527
   <212> PRT
   <213> Bakteriophage T4D
<400> 1
   Met Ser Asn Asn Thr Tyr Gln His Val Ser Asn Glu Ser Arg Tyr Val
   1               5                   10                  15
   Lys Phe Asp Pro Thr Asp Thr Asn Phe Pro Pro Glu Ile Thr Asp Val
               20                  25                  30
   His Ala Ala Ile Ala Ala Ile Ser Pro Ala Gly Val Asn Gly Val Pro
           35                  40                  45
   Asp Ala Ser Ser Thr Thr Lys Gly Ile Leu Phe Leu Ala Thr Glu Gln
       50                  55                  60
   Glu Val Ile Asp Gly Thr Asn Asn Thr Lys Ala Val Thr Pro Ala Thr
   65                  70                  75                  80
   Leu Ala Thr Arg Leu Ser Tyr Pro Asn Ala Thr Glu Ser Val Tyr Gly
                   85                  90                  95
   Leu Thr Arg Tyr Ser Thr Asp Asp Glu Ala Ile Ala Gly Val Asn Asn
               100                 105                 110
   Glu Ser Ser Ile Thr Pro Ala Lys Phe Thr Val Ala Leu Asn Asn Val
   115 120 125
   Phe Glu Thr Arg Val Ser Thr Glu Ser Ser Asn Gly Val Ile Lys Ile
   130 135 140
   Ser Ser Leu Pro Gln Ala Leu Ala Gly Ala Asp Asp Thr Thr Ala Met
   145 150 155 160
   Thr Pro Leu Lys Thr Gln Gln Leu Ala Val Lys Leu Ile Ala Gln Ile
   165 170 175
   Ala Pro Ser Lys Asn Ala Ala Thr Glu Ser Glu Gln Gly Val Ile Gln
   180 185 190
   Leu Ala Thr Val Ala Gln Ala Arg Gln Gly Thr Leu Arg Glu Gly Tyr
   195 200 205
   Ala Ile Ser Pro Tyr Thr Phe Met Asn Ser Thr Ala Thr Glu Glu Tyr
   210 215 220
   Lys Gly Val Ile Lys Leu Gly Thr Gln Ser Glu Val Asn Ser Asn Asn
   225 230 235 240
   Ala Ser Val Ala Val Thr Gly Ala Thr Leu Asn Gly Arg Gly Ser Thr
   245 250 255
   Thr Ser Met Arg Gly Val Val Lys Leu Thr Thr Thr Ala Gly Ser Gln
   260 265 270
   Ser Gly Gly Asp Ala Ser Ser Ala Leu Ala Trp Asn Ala Asp Val Ile
   275 280 285
   His Gln Arg Gly Gly Gln Thr Ile Asn Gly Thr Leu Arg Ile Asn Asn
   290 295 300
   Thr Leu Thr Ile Ala Ser Gly Gly Ala Asn Ile Thr Gly Thr Val Asn
   305 310 315 320
   Met Thr Gly Gly Tyr Ile Gln Gly Lys Arg Val Val Thr Gln Asn Glu
   325 330 335
   Ile Asp Arg Thr Ile Pro Val Gly Ala Ile Met Met Trp Ala Ala Asp
   340 345 350
   Ser Leu Pro Ser Asp Ala Trp Arg Phe Cys His Gly Gly Thr Val Ser
   355 360 365
   Ala Ser Asp Cys Pro Leu Tyr Ala Ser Arg Ile Gly Thr Arg Tyr Gly
   370 375 380
   Gly Ser Ser Ser Asn Pro Gly Leu Pro Asp Met Arg Gly Leu Phe Val
   385 390 395 400
   Arg Gly Ser Gly Arg Gly Ser His Leu Thr Asn Pro Asn Val Asn Gly
   405 410 415
   Asn Asp Gln Phe Gly Lys Pro Arg Leu Gly Val Gly Cys Thr Gly Gly
   420 425 430
   Tyr Val Gly Glu Val Gln Lys Gln Gln Met Ser Tyr His Lys His Ala
   435 440 445
   Gly Gly Phe Gly Glu Tyr Asp Asp Ser Gly Ala Phe Gly Asn Thr Arg
   450 455 460
   Arg Ser Asn Phe Val Gly Thr Arg Lys Gly Leu Asp Trp Asp Asn Arg
   465 470 475 480
   Ser Tyr Phe Thr Asn Asp Gly Tyr Glu Ile Asp Pro Ala Ser Gln Arg
   485 490 495
   Asn Ser Arg Tyr Thr Leu Asn Arg Pro Glu Leu Ile Gly Asn Glu Thr
   500 505 510
   Arg Pro Trp Asn Ile Ser Leu Asn Tyr Ile Ile Lys Val Lys Glu
   515 520 525
<210> 2
   <211> 1578
   <212> DNA
   <213> Bakteriophage T4D
<400> 2
   agtaataata catatcaaca cgtttctaat gaatctcgtt atgtaaaatt tgatcctacc 60
   gatacgaatt ttccaccgga gattactgat gttcacgctg ctatagcagc catttctcct 120
   gctggcgtaa atggagttcc tgatgcatcg tcaacaacaa agggaatttt atttcttgcc 180
   actgaacagg aagttataga tggaactaat aataccaaag cagttacacc agcaacgttg 240
   gcaacaagat tatcatatcc aaacgcaact gaaagtgttt acggattaac aagatattca 300
   accgatgatg aagccattgc cggagttaat aatgaatctt ctataactcc agctaaattt 360
   actgttgctc ttaataatgt ttttgaaact cgtgtttcaa ctgaatcatc aaatggggtt 420
   attaaaattt catctttacc gcaagcattg gcaggtgcag atgatactac tgcaatgact 480
   ccattaaaaa cacaacaatt agctgttaaa ttgattgcgc aaattgctcc ttctaaaaat 540
   gctgctacag aatctgagca aggtgtaatt cagttagcta cagtagcaca ggctcgtcag 600
   ggaactttaa gagaaggata cgcaatttct ccttatacgt ttatgaattc tactgctact 660
   gaagaatata aaggcgtaat taaattagga acgcaatcag aagttaactc gaataatgct 720
   tctgttgcgg ttactggagc aactcttaat ggtcgtggtt ctacgacgtc aatgagaggc 780
   gtagttaaat taactacaac cgccggttca cagagtggag gcgatgcttc atcagcctta 840
   gcttggaatg ctgacgttat ccaccaaaga ggcggtcaaa ctattaatgg aacacttcgc 900
   attaataata cgcttacaat agcttcaggt ggggcaaata ttaccggaac agttaacatg 960
   actggcggtt atattcaagg taaacgcgtc gtaacacaaa atgaaattga tagaactatt 1020
   cctgtcggag ctattatgat gtgggccgct gatagtcttc ctagtgatgc ttggcgtttt 1080
   tgccacggtg gaactgtttc agcgtcagat tgtccattat atgcttctag aattggaaca 1140
   agatatggcg gaagctcatc aaatcctgga ttgcctgaca tgcgcggtct ttttgttcgt 1200
   ggctctggcc gtggctctca tttaacaaat ccaaatgtta atggtaatga ccaatttggt 1260
   aaacctagat taggtgtagg ttgtactggt ggatatgttg gtgaagtaca gaaacaacag 1320
   atgtcttatc ataaacatgc tggtggattt ggtgagtatg atgattctgg ggcattcggt 1380
   aatactcgta gatcaaattt tgttggtaca cgtaaaggac ttgactggga taaccgttca 1440
   tacttcacta atgacgggta tgaaattgac ccagcatcac aacgaaattc cagatataca 1500
   ttaaatcgtc ctgaattaat tggaaatgaa acacgtccat ggaacatttc tttaaactac 1560
   ataattaagg taaaagaa 1578
<210> 3
   <211> 317
   <212> PRT
   <213> Künstliche Sequenz
<220>
   <223> Beschreibung der künstlichen Sequenz: Polypeptid-Fragment
<400> 3
   Leu Ser Tyr Pro Asn Ala Thr Glu Ser Val Tyr Gly Leu Thr Arg Tyr
   1 5 10 15
   Ser Thr Asp Asp Glu Ala Ile Ala Gly Val Asn Asn Glu Ser Ser Ile
   20 25 30
   Thr Pro Ala Lys Phe Thr Val Ala Leu Asn Asn Val Phe Glu Thr Arg
   35 40 45
   Val Ser Thr Glu Ser Ser Asn Gly Val Ile Lys Ile Ser Ser Leu Pro
   50 55 60
   Gln Ala Leu Ala Gly Ala Asp Asp Thr Thr Ala Met Thr Pro Leu Lys
   65 70 75 80
   Thr Gln Gln Leu Ala Val Lys Leu Ile Ala Gln Ile Ala Pro Ser Lys
   85 90 95
   Asn Ala Ala Thr Glu Ser Glu Gln Gly Val Ile Gln Leu Ala Thr Val
   100 105 110
   Ala Gln Ala Arg Gln Gly Thr Leu Arg Glu Gly Tyr Ala Ile Ser Pro
   115 120 125
   Tyr Thr Phe Met Asn Ser Thr Ala Thr Glu Glu Tyr Lys Gly Val Ile
   130 135 140
   Lys Leu Gly Thr Gln Ser Glu Val Asn Ser Asn Asn Ala Ser Val Ala
   145 150 155 160
   Val Thr Gly Ala Thr Leu Asn Gly Arg Gly Ser Thr Thr Ser Met Arg
   165 170 175
   Gly Val Val Lys Leu Thr Thr Thr Ala Gly Ser Gln Ser Gly Gly Asp
   180 185 190
   Ala Ser Ser Ala Leu Ala Trp Asn Ala Asp Val Ile His Gln Arg Gly
   195 200 205
   Gly Gln Thr Ile Asn Gly Thr Leu Arg Ile Asn Asn Thr Leu Thr Ile
   210 215 220
   Ala Ser Gly Gly Ala Asn Ile Thr Gly Thr Val Asn Met Thr Gly Gly
   225 230 235 240
   Tyr Ile Gln Gly Lys Arg Val Val Thr Gln Asn Glu Ile Asp Arg Thr
   245 250 255
   Ile Pro Val Gly Ala Ile Met Met Trp Ala Ala Asp Ser Leu Pro Ser
   260 265 270
   Asp Ala Trp Arg Phe Cys His Gly Gly Thr Val Ser Ala Ser Asp Cys
   275 280 285
   Pro Leu Tyr Ala Ser Arg Ile Gly Thr Arg Tyr Gly Gly Ser Ser Ser
   290 295 300
   Asn Pro Gly Leu Pro Asp Met Arg Gly Leu Phe Val Arg
   305 310 315
<210> 4
   <211> 951
   <212> DNA
   <213> Künstliche Sequenz
<220>
   <223> Beschreibung der künstlichen Sequenz: DNA
<400> 4
   ttatcatatc caaacgcaac tgaaagtgtt tacggattaa caagatattc aaccgatgat 60
   gaagccattg ccggagttaa taatgaatct tctataactc cagctaaatt tactgttgct 120
   cttaataatg tttttgaaac tcgtgtttca actgaatcat caaatggggt tattaaaatt 180
   tcatctttac cgcaagcatt ggcaggtgca gatgatacta ctgcaatgac tccattaaaa 240
   acacaacaat tagctgttaa attgattgcg caaattgctc cttctaaaaa tgctgctaca 300
   gaatctgagc aaggtgtaat tcagttagct acagtagcac aggctcgtca gggaacttta 360
   agagaaggat acgcaatttc tccttatacg tttatgaatt ctactgctac tgaagaatat 420
   aaaggcgtaa ttaaattagg aacgcaatca gaagttaact cgaataatgc ttctgttgcg 480
   gttactggag caactcttaa tggtcgtggt tctacgacgt caatgagagg cgtagttaaa 540
   ttaactacaa ccgccggttc acagagtgga ggcgatgctt catcagcctt agcttggaat 600
   gctgacgtta tccaccaaag aggcggtcaa actattaatg gaacacttcg cattaataat 660
   acgcttacaa tagcttcagg tggggcaaat attaccggaa cagttaacat gactggcggt 720
   tatattcaag gtaaacgcgt cgtaacacaa aatgaaattg atagaactat tcctgtcgga 780
   gctattatga tgtgggccgc tgatagtctt cctagtgatg cttggcgttt ttgccacggt 840
   ggaactgttt cagcgtcaga ttgtccatta tatgcttcta gaattggaac aagatatggc 900
   ggaagctcat caaatcctgg attgcctgac atgcgcggtc tttttgttcg t         951

## Beschreibung

Die vorliegende Erfindung betrifft ein Verfahren zum gleichzeitigen Nachweis verschiedener Bakterien, umfassend die folgenden Schritte: Kopplung von einer oder mehrerer Arten von Bakteriophagenschwanzproteinen an einen Träger, Inkubieren des mit den Bakteriophagenschwanzproteinen gekoppelten Trägers mit einer Probe, gegebenenfalls Entfernen der Probe und der nicht an die Bakteriophagenschwanzproteine gebundenen Bakterien der Probe, In-Kontakt-Bringen der an die Bakteriophagenschwanzproteine gebundenen Bakterien mit die nachzuweisenden Bakterien spezifisch bindenden Bakteriophagen und/oder Bakteriophagenproteinen, Entfernen der nicht an die Bakterien gebundenen Bakteriophagen und/oder Bakteriophagenproteinen, Durchführen der Nachweisreaktion mittels eines an die spezifisch bindenden Bakteriophagen und/oder Bakteriophagenproteine gekoppelten Enzyms oder eines Immunnachweises.

Der schnelle und exakte Nachweis von Bakterien ist unabdingbar zur Kontrolle der Hygiene und Qualität von Rohprodukten und verarbeiteten Nahrungmitteln sowie zur Kontrolle der Hygiene und Qualität von Trink- und Brauchwasser und der Wasserqualität von öffentlichen Bädern. Darüber hinaus dient der Nachweis dem Prozeßmonitoring und -Optimierung, sowie der Qualitätskontrolle in der Umweltanalytik.

Trotz des enormen wirtschaftlichen Interesses sind die zur Zeit verwendeten Testverfahren wie etwa die "BAM"-Methoden (Bacteriological Analytical Manual) sehr zeit-, personal- und ausrüstungsintensiv. Potentielle Krankheitskeime werden sequentiell in selektiven und nicht selektiven Medien angereichert und auf geeigneten Nährböden nachgewiesen. Daran schliesst sich in der Regel eine biochemische und serologische Kontrolle an, so dass das gesamte Verfahren 3 bis 4 Tage beansprucht. Für bestimmte Fragestellungen, z.B. beim Hygienemonitoring kann es notwendig sein, statt einzelner Bakterienspezies ganze Bakteriengruppen nachzuweisen. Dies kann aber auch dann wichtig sein, wenn z.B. der Anteil pathogener Bakterien in einer Gruppe sehr groß ist. Die Tests können nur von mikrobiologisch geschultem Personal in entsprechend ausgestatteten Labors durchgeführt werden. Dies hat zur Folge, dass derzeit selbst große Unternehmen, nur coliforme Bakterien im Haus testen, jedoch alle übrigen Tests, v.a. den Nachweis pathogener Keime, auslagem, Der Coliformen-Nachweis wird im wesentlichen als Hygienemonitoring eingesetzt, wird in der Regel über klassische Nährbodenverfahren geführt und dauert somit mindestens zwei Tage.

Der Nachweis von Bakterien erfolgt aus biologischen Proben zumeist mit Hilfe einer Kombination von Kulturmethoden. Insbesondere zu einer genauen Charakterisierung wird darüberhinaus auf die Technik der Phagen-Typisierung (engl.: "phage-typing") zurückgegriffen. Dabei werden Kulturmethoden mit der Empfindlichkeit von Bakterien gegen Typisierungs-Bakteriophagen gekoppelt. Bakteriophagen werden bereits seit etwa 80 Jahren im Phage-typing eingesetzt, bei dem mit Hilfe von speziellen Klassifizierungs-Phagen Bakterienstämme anhand standardisierter Schemata bestimmt werden können. Es wird ein dichter Bakterienrasen auf einer Agarplatte der zu untersuchenden Probe, der durch Isolierung einer Einzelkolonie und anschliessender Vermehrung derselben gewonnen wurde, mit Suspension aus Bakteriophagen in Softagar überschichtet. Das Ergebnis wird nach Übernachtinkubation bei der optimalen Wachstumstemperatur der Bakterien, die üblicherweise zumeist 37°C beträgt, durch Auszählen der Plaques und der Kontrolle der Plaquemorphologie erhalten.

Neuere alternative Methoden, die ohne Nährboden auskommen, stützen sich vor allem auf DNAsowie Antikörpertechnologien. Im erstgenannten Ansatz werden die Bakterien an Filter gebunden und die Bakterien-DNA mit geeigneten DNA-Sonden, die ein Messsignal geben, hybridisiert oder die amplifizierte Bakterien-DNA wird mittels PCR direkt ansequenziert.

So lehrt zum Beispiel die Veröffentlichung von Bennett et al., Journal of Applied Microbiology 1977, 83 259-265, dass Salmonellen mittels Salmonellen-spezifischen Bakteriophagen, die auf einem Träger immobilisiert wurden, aus einer Probe angereichert und mittels PCR nachgewiesen werden können.

In der WO 93/17129 wird ein Verfahren offenbart, mit dem Bakterien in einer Probe nachgewiesen werden, indem ein Bakteriophage an das nachzuweisende Bakterium bindet. Die Bakteriophagen sind mit einem ersten Teil eines spezifischen Bindungspaares verbunden. Der zweite Teil des Bindungspaares ist mit einem Träger verbunden. Der Nachweis der spezifischen Bakterien erfolgt dann über die Bindung des ersten mit dem zweiten Teil des Bindungspaares, so dass die Bakterien dann über einen Marker nachgewiesen werden, der wiederum an den Bakteriophagen oder den Träger gekoppelt ist.

In der ELISA-Technologie werden Bakterien oder Bakterienbestandteile mit Hilfe von monoklonalen Antikörpern gebunden und über enzymatische Sonden detektiert, die an einen sekundären Antikörper gekoppelt sind. Beide Methoden erfordern jedoch ebenfalls einen hohen technischen Aufwand und es ist für die benötigten 10⁴ bis 10⁶ Zellen pro ml ein Anreicherungsschritt vorzuschalten. Damit fällt die Zeitersparnis gegenüber herkömmlichen Methoden so gering aus, dass die genannten Methoden kaum Anwendung finden.

Die Verwendung von Bakteriophagen als "Biosorbentien" für die Bindung der nachzuweisenden Bakterien hat gegenüber den neueren Verfahren einige entscheidende Vorteile. Die Phagen-Bakterien-Systeme sind in der Natur über lange Zeiträume evolviert, so dass die Phagen ihre Wirtsbakterien hochspezifisch und mit hoher Bindungsaffinität selbst in komplexen Umgebungen, wie sie in der Natur, aber auch in Nahrungsmitteln vorliegen, erkennen. Zeitaufwändige Anreicherungskulturen, bei denen häufig auch die für den Nachweis unerwünschten Keime wie z.B. Impfkulturen mit angereichert werden, werden durch das hier beschriebene neuartige Verfahren weitgehend unnötig. Nachteilig der bisher beschriebenen Phagen-basierten Bakteriennachweissysteme ist jedoch, daß sie meist einzelne Bakterienarten hochspezifisch erkennen und somit für einen Nachweis von Bakteriengruppen, -familien oder mehrere Bakterienarten nicht verwendet werden können.

Daher liegt der Erfindung die Aufgabe zu Grunde, ein Nachweisverfahren für Bakterien bereitzustellen, mit dem gleichzeitig verschiedene Bakterienarten nachgewiesen werden können. Eine weitere Aufgabe besteht in der Bereitstellung von Bakteriophagenschwanzproteinen, die eine für das Nachweisverfahren vorteilhaftere Bakterienbindung aufweisen.

Die Aufgabe wird durch den in den Patentansprüchen definierten Gegenstand gelöst.

Die Erfindung wird durch die folgenden Figuren erläutert.

Fis. 1A und B zeigt schematisch einen gruppenspezifischen Nachweis. (1A): Das Bakteriophagenschwanzproiein (1) wird über Biotin (2) an Streptavidin (3) gebunden, das kovalent mit der Matrix (4) verbunden ist. Bakterien aus der zu erkennenden Gruppe (5) werden spezifisch an die Bakteriophagenschwanzproteine (1) gebunden. (1B): Nach einem Waschschritt werden die gebundenen Bakterien (5) mittels desselben Bakteriophagenschwanzproteins (1), das jedoch diesmal an ein Enzym (6) z. B. Peroxidase gekoppelt ist, detektiert, hv bedeutet Signalentwicklung die z.B. per Absorption verfolgt werden kann.

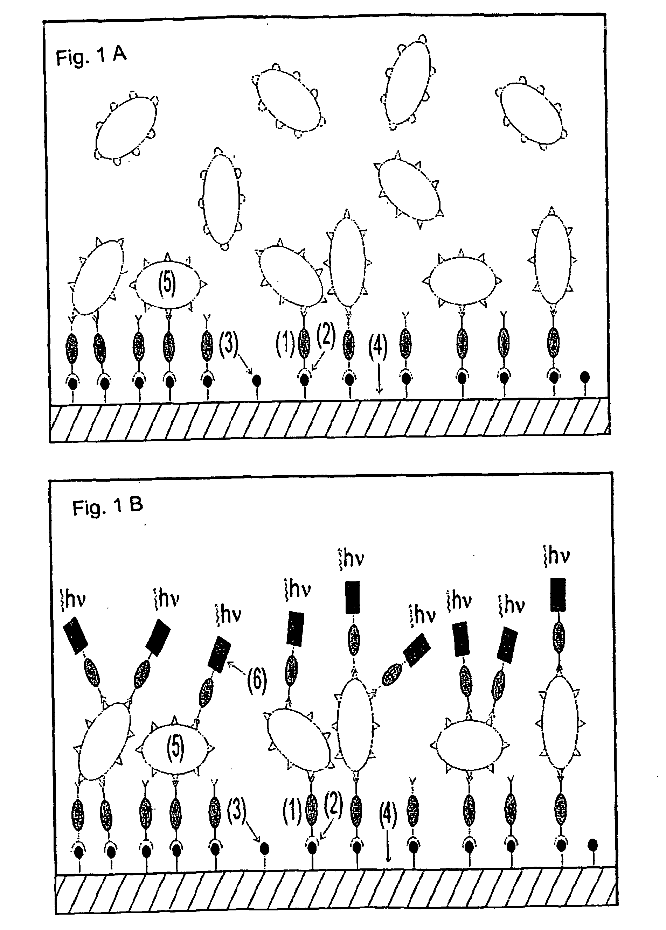

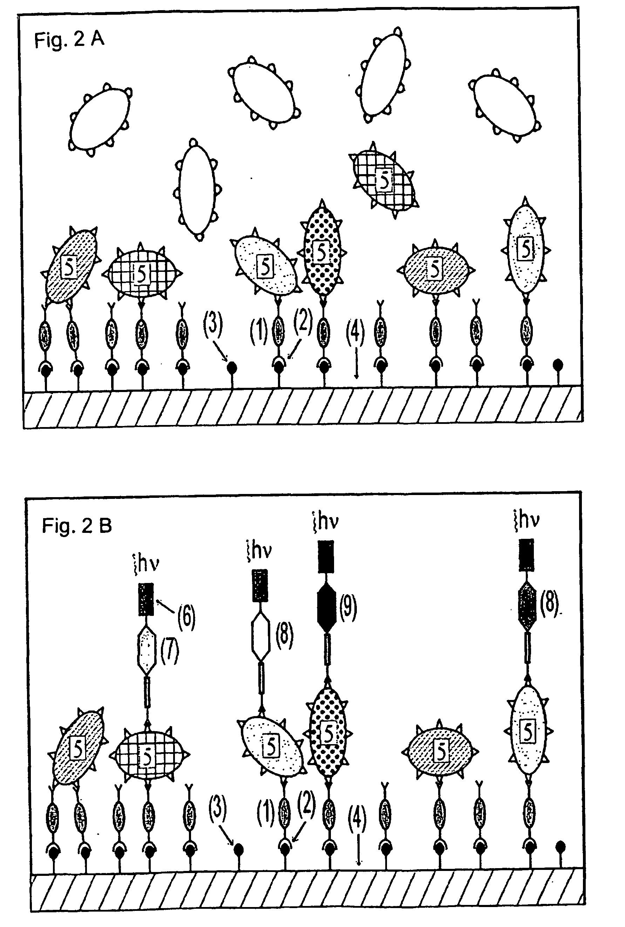

Fig. 2A und B zeigt schematisch einen gruppenspezifischen Nachweis. (2A): Das Bakteriophagenschwanzprotein (1) wird über Biotin (2) an Streptavidin (3) gebunden, das kovalent mit der Matrix (4) verbunden ist. Bakterien aus der zu erkennenden Gruppe (5) werden spezifisch an die Bakteriophagenschwanzproteine (1) gebunden. (2B): Nach einem Waschschritt, werden die gebundenen Bakterien (5) mittels stammspezifischer Bakteriophagen (7, 8, 9), die mit einem Enzym (6) z. B. Peroxidase gekoppelt sind, detektiert, hv bedeutet Signalentwicklung die z.B. per Absorption verfolgt werden kann.

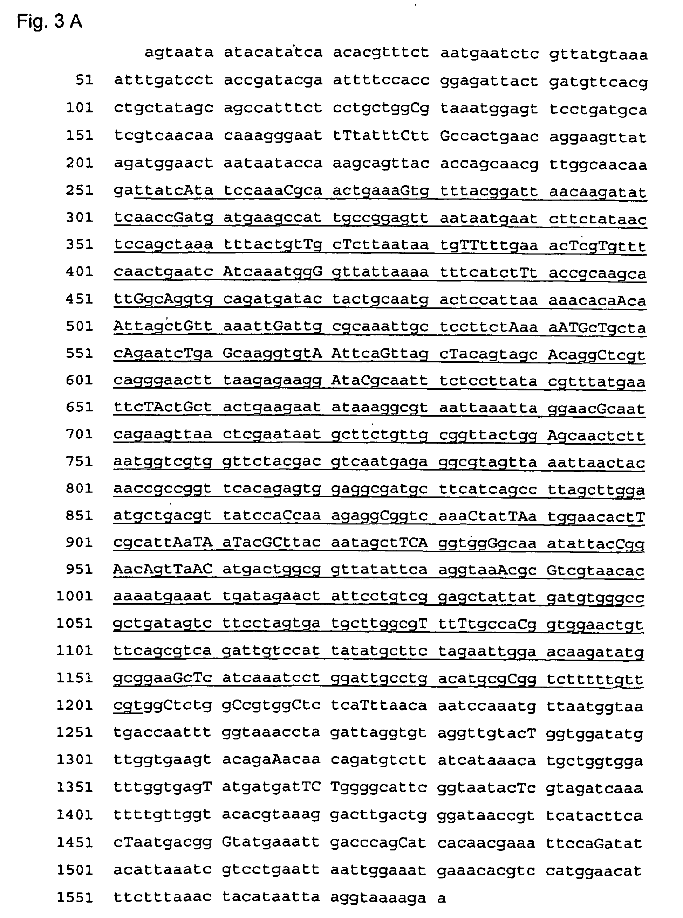

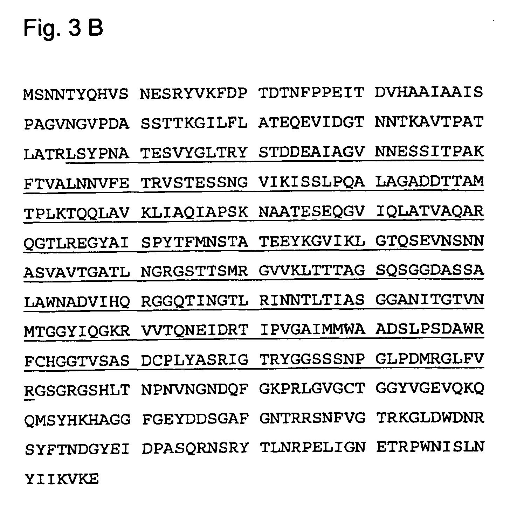

Fig. 3 A zeigt die DNA-Sequenz der kurzen Schwanzfaser (p12) des Phagenstammes T4D (SEQ ID NO:2). Fig. 3 B zeigt die Aminosäuresequenz dieser Variante (SEQ ID NO:1): Die Nukleinsäuresequenz gemäß SEQ ID NO:4 und die Aminosäuresequenz gemäß SEQ ID NO:3 sind unterstrichen. Großbuchstaben in der Nukleinsäuresequenz stellen Basenaustausche gegenüber der Wildtyp-Sequenz T4 p 12 (Swiss-Prot. accession number P 10930) dar.

Der hier verwendete Ausdruck "coliforme" bezeichnet eine Untergruppe der Enterobakterien, die sich dadurch auszeichnen, dass sie Laktose verwerten bzw. das Enzym β-Galaktosidase exprimieren.

Der hier verwendete Ausdruck "Bakteriengruppe" bezeichnet eine Zusammenfassung bestimmter Bakterien, die innerhalb einer Familie, oder einer Gattung, oder einer Art etc., aber auch übergreifend sein kann.

Der hier verwendete Ausdruck "Derivat" bezeichnet Nukleinsäure- oder Aminosäuresequenzen, die gegenüber der Vergleichssequenz Modifikationen in Form von einer oder mehreren Deletionen, Substitutionen, Additionen, Insertionen und/oder Inversionen aufweisen.

Der hier verwendete Ausdruck "Fragmente" bezeichnet Teile von Aminosäuresequenzen sowie die für diese Aminosäuresequenzen kodierenden Nukleinsäuresequenzen, solange sie die biologische Funktion der erfindungsgemäßen Aminosäuresequenzen aufweisen.

Ein Aspekt der vorliegenden Erfindung besteht daher in der Bereitstellung eines Verfahrens zum gleichzeitigen Nachweis verschiedener Bakterien, umfassend die folgenden Schritte: Kopplung von einer oder mehrerer Arten von Bakteriophagenschwanzproteinen an einen Träger, Inkubieren des mit den Bakteriophagenschwanzproteinen gekoppelten Trägers mit einer Probe, gegebenenfalls Entfernen der Probe und der nicht an die Bakteriophagenschwanzproteine gebundenen Bakterien der Probe, In-Kontakt-Bringen der an die Bakteriophagenschwanzproteine gebundenen Bakterien mit die nachzuweisenden .Bakterien spezifisch bindenden Bakteriophagen und/oder Bakteriophagenproteinen, Entfernen der nicht an die Bakterien gebundenen Bakteriophagen und/oder Bakteriophagenproteine, Durchführen der Nachweisreaktion mittels eines an die spezifisch bindenden Bakteriophagen und/oder Bakteriophagenproteine gekoppelten Enzyms oder eines Immunnachweises. Alternativ kann der Nachweis über (bakterien)zelleigene Enzyme (z.B. β-Galactosidase bei Coliformen) oder auch andere Zellbestandteile wie Nukleinsäuren, Proteine, Lipide oder Zuckeranteile geführt werden.

Für das erfindungsgemäße Verfahren werden Bakteriophagenschwanzproteine verwendet, die für die gewünschten nachzuweisenden Bakterien spezifisch sind. Die Bakteriophagenschwanzproteine sind dabei für mehrere Bakterienarten spezifisch. Die nachzuweisenden Bakterien können z.B. ganze Bakterienfamilien, eine oder mehrere ganze Bakteriengattungen, bestimmte Bakterienarten aus einer Bakteriengattung, bestimmte Bakterienarten aus mehreren Bakteriengattungen oder dergleichen sein. Die mit dem erfindungsgemäßen Verfahren nachzuweisenden Bakterien können zum Beispiel aus der Familie der Enterobakteriaceae stammen. Vorzugsweise werden Bakterien aus der Gattung *Escherichia, Salmonella, Shigella, Klebsiella, Enterobacter, Citrobacter, Proteus, Serratia, Morganella, Providencia, Yersinia, Hafnia* und/oder *Edwardsiella* nachgewiesen, insbesondere bevorzugt aus *Escherichia, Salmonella, Shigella, Klebsiella, Enterobacter* und/oder *Citrobacter.* Ferner vorzugsweise sind nachzuweisenden Bakterien die Arten *Escherichia spec., Salmonella spec., Shigella spec., Klebsiella spec., Enterobacter spec., Citrobacter spec.. Proteus spec., Serratia spec. Morganella spec., Providencia spec., Yersinia spec., Hafnia spec., Edwardsiella spec,* und/oder *Pseudomonas spec.*

Beispielsweise ist es für den Anwender z. B. in der Milch- oder Getränkeindustrie irrelevant, Bakterienkontaminationen serologisch und stammspezifisch zu charakterisieren. Wichtig ist nur die generelle Information, ob z. B. im Produktionsablauf Fremdkeime in die Anlage eingetreten sind oder Entkeimungsprozesse erfolgreich waren. Coliforme Bakterien dienen dabei in der Regel als Hygieneindikator. So ist eine besondere Ausführungsform des erfindungsgemäßen Verfahrens der Nachweis von coliformen Bakterien, insbesondere der Gattungen *Escherichia, Salmonella, Shigella, Klebsiella, Emerobacter, Citrobacter. Proteus, Serratia, Morganella*, *Providencia, Yersinia, Hafnia* und/oder *Edwardsiella.*

Welche Bakteriophagenschwanzproteine verwendet werden, hängt davon ab, welche Bakterienarten, -gruppen oder -familien nachgewiesen werden sollen. Für den erfindungsgemäßen gruppenspezifischen Nachweis werden solche Bakteriophagenschwanzproteine eingesetzt, die gruppenspezifische Rezeptoren erkennen und binden. Dabei kann jedes Phagenschwanzprotein verwendet werden, das ein entsprechendes Erkennungsspektrum aufweist. Bevorzugt sind Phagenschwanzproteine aus der Familie der Myoviridae, der Podoviridae und Siphoviridae, insbesondere kurze Phagenschwanzproteine, insbesondere die kurzen Phagenschwanzproteine der "geradzahligen T-Phagen" z.B. von T4, T2 oder K3, insbesondere das Bakteriophagenschwanzprotein p12 aus T4, p12 aus T2 (GenBank Accession Nummer X56555), p12 aus K3 (vgl. Burda et al., 2000, Biol. Chem., Vol. 381, pp. 255- 258,) oder die Bakteriophagenschwanzproteine der Phagen Felix 01, P1 oder PB1. Dabei binden zum Beispiel die kurzen Bakteriophagenschwanzproteine der Phagen T4 (p12) und von P1 an Coliforme, das kurze Phagenschwanzprotein von Felix 01 an Salmonellen und das kurze Phagenschwanzprotein von PB1 an Pseudomonaden. Beispielsweise erkennt der *Salmonellenphage Felix 01* (F01) bis zu 99 % aller Salmonellen und kann für einen gruppenspezifischen Salmonellen-Nachweis verwendet werden. Die Bakteriophagenschwanzproteine des PB1 können für einen gruppenspezifischen Pseudomonaden-Nachweis verwendet werden.

Wie die Bakteriophagenschwanzproteine an die einzelnen Bakterien binden soll hier beispielhaft an den "geradzahligen T-Phagen" z.B. T4, T2, K3, verdeutlicht werden. In dieser Gruppe gibt es auf Wirtsseite zwei Komponenten, die von den Phagen erkannt werden: Erstens ein Oberflächenprotein, das spezifisch für individuelle Phagen ist, zweitens das Lipopolysaccharid (LPS), das alle gram-negativen Bakterien in abgewandelter Form besitzen. Die langen Schwanzfasern der "geradzahligen T-Phagen" spielen eine Rolle bei der spezifischen Erkennung der Wirtsbakterien, während das LPS als Rezeptor für die kurzen Schwanzfasern dient. Beim E. coli Phagen T4 ist bekannt, dass die durch die langen Schwanzfasern vermittelte spezifische Interaktion mit dem Wirtsbakterium irreversibel wird, sobald auch die kurzen Schwanzfasern an die Bakterienoberfläche gebunden haben. Die kurze Schwanzfaser ist nicht verantwortlich für die genaue Spezifität innerhalb der Wirtsbakteriengruppe und kann deshalb zwischen den verschiedenen "geradzahligen T-Phagen" ausgetauscht werden

Die kurzen Schwanzfasern sind zusammen mit den ,Tail Pins' am sogenannten ,Pinning'-Schritt beteiligt, bei dem die irreversible Bindung des Phagen an LPS auf der Wirtsoberfläche erfolgt. p12 benötigt für die Bindung an LPS eine Struktur von Heptosezuckern mit mindestens einer α-gebundenen Glucose, wie sie in der inneren Kernzone der Zellwand von Enterobakterien zu finden ist. Unterschiedliche Phosphorylierungen der Zucker, wie sie häufig auftreten, das Vorhandensein von Substitutionen mit Pyrophosphatidylethanolamin oder auch die Verzweigungen der Heptoseregion scheinen keine Rolle zu spielen. Obwohl sich die Lipopolysaccharide der einzelnen Bakterienstämme in den O-spezifischen Seitenketten, - was für die serologische Typisierung genutzt wird -, aber auch noch in den äusseren Kernregionen sehr stark unterscheiden, ist die Varianz im inneren Kernbereich sehr viel weniger ausgeprägt. Alle enterobakteriellen Kemregionen weisen beispielsweise folgende zwei Zucker auf: 3-Desoxy-D-manno-octuronsäure (Kdo) und L-Glycero-D-manno-heptopyranose (Hep), die folgendes charakteristische Oligosaccharid in der inneren Kernzone ausbilden:

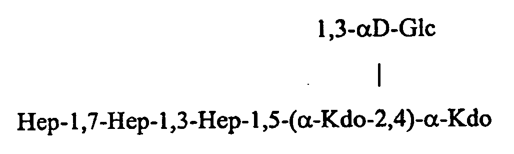

Die mittlere Heptose ist in der Regel an 03 substituiert und trägt unter anderem bei Escherichia coli, sowie bei Salmonella enterica, Shigella spp., Hafnia spp., Citrobacter spp. und Erwinia spp. eine 1,3-gebundene αD-Glucose. Klebsiella, Proteus und Yersinia weisen an dieser Position entweder Galactose oder ein N-Acetylfucosamin auf und werden von p12 nicht erkannt. Klebsiella wird jedoch z.B. vom kurzen Bakteriophagenschwanzprotein des Phagen P1 erkannt.

Bakteriophagenschwanzproteine, deren Derivate, Varianten oder Fragmente können leicht rekombinant in großen Mengen produziert und isoliert werden. Für das erfindungsgemäße Verfahren können jedoch nicht nur die natürlicherweise vorkommenden Bakteriophagenschwanzproteine verwendet werden, sondern auch deren Varianten. Der Ausdruck "Varianten" bedeutet im Sinne der vorliegenden Erfindung, dass die Bakteriophagenschwanzproteine eine veränderte Aminosäuresequenz gegenüber der Wildtypsequenz aufweisen. Natürlicherweise vorkommende Varianten können durch übliche Screening-Verfahren gefunden werden. Zu den Varianten zählen ferner auch synthetisch hergestellte Bakteriophagenschwanzproteine. Die synthetisch hergestellten Polypeptide können zum Beispiel durch gezielte oder zufällige (random) Mutagenese hergestellt werden. Durch die Mutagenese können Modifikationen eingeführt werden, die Aminosäureadditionen, -deletionen, - substitutionen, -inversionen oder -insertionen sein können. Die Bakteriophagenschwanzproteine können ferner chemische Modifikation, wie z.B. Methylierungen oder Acetylierungen aufweisen. Die Modifikationen können eine veränderte insbesondere eine verbesserte Wirtsspezifität und auch eine verbesserte Bindungseigenschaft an die Matrix bewirken, z.B. die Bindung der Bakterien an die isolierten Phagenproteine zu erhöhen oder irreversibel zu mächen, um die Waschmöglichkeiten zu verbessern.

Ferner können auch zwei oder mehrere Bakteriophagenschwanzproteine in einem Nachweisverfahren eingesetzt werden, wenn die nachzuweisenden Bakterien nicht durch ein einziges Bakteriophagenschwanzprotein nachgewiesen werden können. Beispielsweise können die kurzen Bakteriophagenschwanzproteine der Phagen T4 (p12) und P1 verwendet werden, die ein geringfügig unterschiedliches Wirtsspektrum besitzen, um gleichzeitig neben den von beiden Bakteriophagenschwanzproteinen erkannten Bakteriengattungen *Escherichia, Salmonella, Shigella, Citrobacter* und *Erwinia,* die nur von T4 p12 erkannte Gattung Hafnia, und die nur von dem kurzen Bakteriophagenschwanzprotein des Phagen P1 erkannten Gattungen *Klebsiella* und *Enterobacter* nachzuweisen. Ferner kann es für den Nachweis von Salmonellen vorteilhaft sein, das kurze Phagenschwanzprotein von Felix 01 mit anderen Bakteriophagenschwanzproteinen zu verwenden, da derzeit 2500 Salmonellenserovare (Subtypen von Bakterien, die an Hand serologischer Merkmale z.B. LPS-Variationen im O-Antigen unterschieden werden können) beschrieben sind. Welche Bakteriophagenschwanzproteine für den Nachweis verwendet werden, hängt letztendlich davon ab, welche Bakterienarten, -gruppen oder -familien nachgewiesen werden sollen.

Vorzugsweise ist das Bakteriophagenschwanzprotein eine Variante des Wildtyp p12-Proteins gemäß SEQ ID NO:1, besonders bevorzugt gemäß SEQ ID NO:3. Die Erfindung betrifft ferner die Nukleinsäuresequenzen gemäß SEQ ID NO:2 und 4.

SEQ ID NO: 1 stellt die Aminosäuresequenz der natürlicherweise vorkommenden p12-Variante, nämlich T4D p12, dar. Dieses Bakteriophagenschwanzprotein weist z.B. eine sehr hohe Stabilität gegenüber Hitze (Denaturierungsmittelpunkt: 78°C) und chemischen Denaturierungsmitteln auf (Burda et al., 2000, Biol. Chem., Vol. 381, pp. 255 - 258).

Überraschenderweise wurde gefunden, daß ein Protease-resistentes Fragment dieser p12-Variante gegenüber den kompletten p12 Proteinen etwa 10-fach fester an Bakterienzellen bindet. Ferner wird durch den Proteaseverdau der temperatursensitive N-Terminus der kompletten p12-Varianten abgebaut, so daß das Protease-resistente Fragment unter anderem eine größere Langzeitstabilität aufweist. Die Aminosäuresequenz des Protease-resistenten Fragments ist in SEQ ID NO:3, die Nukleinsäuresequenz in SEQ ID NO:4 aufgeführt. Ein Aspekt der vorliegenden Erfindung betrifft somit die Aminosäuresequenz gemäß SEQ ID NO:3 oder deren Derivate, und die Nukleinsäuresequenz gemäß SEQ ID NO:4 oder deren Derivate. Ein weiterer Aspekt der vorliegenden Erfindung betrifft ferner Fragmente und deren Derivate der SEQ ID NO:1, vorzugsweise mit der Sequenz der Aminosäurereste 1-500, 1-450, 40-500, 40-450, 270-400, 270-390, 280-400 und 280-390, insbesondere bevorzugt das Fragment gemäß SEQ ID NO:3, sowie die Nukleinsäuresequenzen kodierend die jeweiligen Fragmente und Derivate.

Ein weiterer Aspekt der vorliegenden Erfindung betrifft poly- und monoklonale Antikörper gegen die Aminosäuresequenz gemäß SEQ ID NO:3.

Für den erfindungsgemäßen Nachweis werden die Bakteriophagenschwanzproteine auf geeigneten Trägerstrukturen, z.B. Mikrotiterplatten, Teststreifen, Objektträgern, Wafern, Filtermaterialien oder Durchflußzellkammern, immobilisiert. Die Trägerstrukturen können z.B. aus Polystyrol, Polypropylen, Polycarbonat, PMMA, Celluloseacetat, Nitrozellulose, Glas, Siliziumwafer bestehen. Die Immobilisierung kann z.B. durch Adsorption oder kovalente Bindung erreicht werden. Wichtig hierbei ist eine funktionelle Immobilisierung, d.h. Bakteriophagenschwanzproteine verfügen trotz Bindung an das Trägermaterial über für Bakterien zugängliche Strukturen.

Zur Unterdrückung einer unspezifischen Reaktion der zu untersuchenden Bakterien mit dem Trägermaterial kann eine Blockierung mit Rinderserumalbumin oder Tween 20 oder ähnlichen aus dem ELISA-Bereich bekannten Substanzen z.B. Milchpulver durchgeführt werden. Ferner können zur Erhöhung der Effizienz der Adsorption die Trägersysteme mit geeigneten Proteinen (z.B. spezifische Antikörper gegen Phagenproteine oder unspezifische Proteine, wie z. B. Rinderserumalbumin), Peptiden, Sacchariden (z.B. Mono-, Oligo- oder Polysaccharide) oder Detergentien (z.B. Tween 20 oder Octylglukosid) vorbeschichtet werden. Diese Beschichtungen können zum Beispiel entweder über Nacht bei einer Temperatur im Bereich von 4-20°C oder in 2-4 h bei 30-65°C durchgeführt werden. Anschließend wird die überschüssige Flüssigkeit abgenommen und die Trägerstruktur bei etwa 60-70°C getrocknet. Die Basisbeschichtung soll zum einen eine Adsorption der Bakteriophagenschwanzproteine gewährleisten und zum anderen eine unspezifische Adsorption der Testbakterien an die Trägerstuktur verhindern, um die Testeffizienz zu erhöhen.

Im Anschluß an die Basisbeschichtung werden die Bakteriophagenschwanzproteine aufgebracht. Dazu wird eine wäßrige gepufferte Lösung der Bakteriophagenschwanzproteine auf die vorbehandelte Trägerstruktur aufgetragen. Nach der Adsorption zum Beispiel bei 4-20°C über Nacht oder bei 30-65°C für 2-4 h wird die Beschichtungslösung abgenommen und die Trägerstruktur wie oben beschrieben getrocknet. Zu einer Erhöhung der Beschichtungseffizienz kann nachträglich eine kovalente Fixierung der Bakteriophagenschwanzproteine mit chemischen Crosslinkem, wie z.B. Glutaraldehyd, durchgeführt werden.

Die Immobilisierung der Bakteriophagenschwanzproteine an das Trägermaterial mittels Adsorption kann durch Inkubation einer Phagenlösung in wässrigem Puffer, z.B. 100mM Tris pH 7.3 oder 100mM Natriumphosphat pH 7.5, über mehrere Stunden oder über Nacht bei 5°C bis 45°C, vorzugsweise bei 15°C bis 37°C, besonders bevorzugt bei 20°C bis 37°C, insbesondere bevorzugt bei RT, durchgeführt werden.

Ferner müssen die Bakteriophagenschwanzproteine nicht direkt auf dem Träger immobilisiert werden, sondern können an Polypeptide binden, die ihrerseits auf dem Träger immobilisiert wurden. Diese Polypeptide können für die Bakteriophagenschwanzproteine spezifische Antikörper, Lektine, Rezeptoren oder Anticaline sein. Ferner können die Bakteriophagenschwanzproteine mit niedermolekularen Substanzen z.B. Biotin verknüpft sein, um über diese niedermolekularen Substanzen an Polypeptide z. B. Streptavidin zu binden, die ihrerseits auf dem Träger immobilisiert wurden. Die anderen Polypeptide können dabei, wie vorstehend für die Bakteriophagenschwanzproteine beschrieben, an den Träger binden.

Bei der kovalenten Kopplung können die Bakteriophagenschwanzproteine z.B. über primäre Aminogruppen oder Carboxylgruppen an bereits vom Hersteller aktivierte Trägermaterialien, z.B. Mikrotiterplatten von Nunc, Xenobind oder Costar, über Standardbedingungen, z.B. -NH₂ über Cyanurylchlorid (Russian Chemical Rev., 1964, 33: 92-103), oder -COO- über EDC (1-Ethyl-3'[3'Dimethylaminopropyl]carbodiimid) (Anal. Biochem. 1990, 185: 131-135) gekoppelt werden. Darüberhinaus können die Trägermaterialien mit geeigneten Verfahren direkt aktiviert werden. Eine Möglichkeit, die wegen der Anwendbarkeit für ein breites Spektrum an Trägermaterialien bevorzugt wird, ist die Silanisierung des Trägermaterials. Beispielsweise kann die Silanisierung bei Polystyrol durch Flammpyrolyse durchgeführt werden. Anschliessend werden geeignete Haftvermittler aufgetragen, die eine Kopplung über z.B. primäre Aminogruppen oder Carboxylgruppen ermöglichen.

Zur Bindung der zu untersuchenden Bakterien an die immobilisierten Bakteriophagenschwanzproteine wird die Untersuchungsprobe in wäßriger Form mit den Bakteriophagenschwanzproteine in Kontakt gebracht und inkubiert. Die Inkubation erfolgt bei einer Temperatur im Bereich von 4° bis 90°C, vorzugsweise bei einer Temperatur im Bereich von 4° bis 45°C, besonders bevorzugt bei einer Temperatur im Bereich von 15° bis 37°C, insbesonders bevorzugt bei einer Temperatur im Bereich von 20° bis 37°C, insbesondere bei RT, für bis zu 6 Stunden, vorzugsweise bis zu 4 Stunden, besonders bevorzugt 2 Stunden, insbesondere 1 Stunde, insbesondere bevorzugt 1 bis 20 Minuten. Beispielsweise kann die Inkubation 2 bis 120 min bei 4° bis 37°C, bevorzugt für 20 bis 30 min bei 25°C oder 37°C, besonders bevorzugt für 35 min bei 37°C erfolgen. Nach der spezifischen Erkennung und fester Bindung der Bakterien kann unspezifisch gebundenes Material durch Waschen mit wässrigen Puffer, z.B. mit PBS oder PBS-Tween, vorzugsweise bei neutralem pH-Wert, z.B. bei 50 mM Natriumphosphat, pH 7.0 abgetrennt werden. Den verwendeten Puffern können gegebenenfalls zur Erhöhung der Wasch-Effizienz Detergenzien, z.B. Tween 20, Triton X 100 oder chaotrope Agentien, z.B. Guanidiniumhydrochlorid oder Harnstoff, zugesetzt werden. Dieser Waschschritt kann abhängig vom Probenmaterial mehrmals wiederholt werden.

Für den Nachweis der an die Baktenophagenschwanzproteine gebundenen Bakterien werden Bakteriophagen oder Bakteriophagenschwanzproteine verwendet. Spezifische Bakteriophagen oder eine Kombination verschiedener Bakteriophagen können für den Nachweis verwendet werden, wenn eine oder mehrere Bakterienarten spezifisch nachgewiesen werden sollen. Beispielsweise sind die in einer Probe vorhandenen coliformen Bakterien durch die immobilisierten Bakteriophagenschwanzproteine gebunden worden. Mittels spezifischer Bakteriophagen können dann einzelne Bakterienarten oder mittels einer Kombination verschiedener Bakteriophagen können mehrere Bakterienarten nachgewiesen werden. Besonders bevorzugt werden die Bakteriophagenschwanzproteine verwendet, die an die Matrix immobilisiert wurden, um die nachzuweisenden Bakterien zu binden. Für den Nachweis ist an die Antikörper, Bakteriophagen oder Bakteriophagenschwanzproteine eine Markierung gekoppelt, z.B. FITC, Peroxidase oder alkalische Phosphatase, wobei die Signalentwicklung der Markierung nach Zugabe eines Substrats photometrisch verfolgt werden kann. Die Gene für die Bakteriophagenschwanzproteine können in geeignete Expressionsvektoren kloniert und auf diese Weise je nach Anwendung zusätzlich modifiziert werden z.B. durch die Fusion mit einem Nachweisenzym.

Nachgewiesen werden je nach Bedarf z.B. Fluoreszenz, Lumineszenz, Absorption oder Circulardichroismus, Leitfähigkeit oder Kapazitätsänderungen der jeweiligen Proben in den entsprechenden Standardmeßgeräten. Für eine exakte Konzentrationsbestimmung der Bakterien kann eine Eichgerade mit entsprechenden Standardmolekülen durchgeführt werden.

Der Nachweis der an Bakteriophagenschwanzproteine gebundenen Bakterien kann ferner durch die Verwendung eines colorimetrischen Tests durchgeführt werden. Dabei wird z.B. NADH, β-Galactosidaseaktivität oder anorganisches Phosphat nachgewiesen. Diese Tests ermöglichen den Nachweis von mindestens 10⁴ Zellen pro ml, durch Verwendung von Fluoreszenzfarbstoffen kann die Sensititvität auf 10² bis 10³ Zellen pro ml verbessert werden.

Die colorimetrischen Tests können für alle zu untersuchenden Bakterien gleich, aber auch für bestimmte Bakterien/Bakteriophagenschwanzprotein-Kombinationen spezifisch sein. Die Auswahl der Markierung z.B. des eingesetzten Enzyms oder Fluoreszenzmarkers kann an die jeweils getestete Bakteriengattung oder die Bakterienarten angepasst werden.

Ein weiterer Aspekt der Erfindung betrifft somit einen Kit zum Nachweis von Bakterien, umfassend einen Träger mit immobilisierten Bakteriophagenschwanzproteinen und die für den Nachweis der gebundenen Bakterien notwendigen Lösungen mit den Testreagentien. Die Träger können alle vorstehend beschriebenen Träger sein, an die die Bakteriophagenschwanzproteine wie vorstehend beschrieben immobilisiert sind. Die Lösungen mit den Testreagentien entsprechen ebenfalls den Substanzen, die für den Nachweis der Bakterien im erfindungsgemäßen Verfahren beschrieben sind. Der Kit kann ferner gegebenenfalls Waschlösungen, sowie für den Aufschluß der Bakterienmembran benötigte Enzyme oder Detergenzien enthalten.

Die folgenden Beispiele erläutern die Erfindung und sind nicht als einschränkend aufzufassen. Sofern nicht anders angegeben, wurden molekularbiologische Standardmethoden verwendet, wie z.B. von Sambrook et al., 1989, Molecular cloning: A Laboratory Manual 2. Auflage, Cold Spring Harbor Laboratory Press, Cold Spring Harbor, New York, beschrieben.

### Beispiel 1: Isolierung und Klonierung von T4D p12, K3 p12 und T2 p12

T4D p12, K3 p12 und T2 p12 wurden wie in Burda et al., 2000, Biol. Chem., Vol. 381, pp. 255-258, beschrieben isoliert und kloniert. T4D p12 wurde entsprechend aufgereinigt und mit Freud'schem Adjuvanz zur Immunisierung von Kaninchen eingesetzt. Das erhaltene polyklonale Antiserum erkannte sowohl das T4D p12 als auch die Protease-resistente Domäne.

### Beispiel 2: Protease-Verdau von T4D p12 und rekombinante Herstellung

Durch Protease-Verdau bei verschiedenen Temperaturen (vgl. Chen & King, 1991, Danner et al., 1993) wurde die Protease-resistente Domäne von p12 erhalten und anschließend durch eine übliche Gelfiltrationschromatographie gereinigt. Der N-Terminus wurde per Edman-Abbau bestimmt und durch Massenspektrometrie wurde der C-Tenminus analysiert. Für die DNA-Sequenz der erhaltenen Domäne wurden die entständigen Primer mit einem Start- und einem Stopkodon entworfen. In einer üblichen PCR mit der cDNA der T4D p12 Variante wurde cDNA der Protease-resistenten Domäne amplifiziert und anschließend in einen Vektor kloniert. Diese Mutante wurde anschliessend in E.coli BL21 DE3 als natives Trimer exprimiert und durch EDTA-Extraktion (vgl. Burda & Miller, 1999, Eur. J. Biochem. 265, 771-778) aus der Membranfraktion isoliert, wobei die Extraktionen in Gegenwart von mindestens 100mM EDTA durchgeführt wurden.

### Beispiel 3: Gruppenspezifischer Nachweis über die bakteneneigene β-Galaktosidase

Dazu wurde p12 (300ng'well in 150 µl 100 mM PBS (4 mM KH₂PO₄, 16 mM Na₂PO₄, 115 mM NaCl); 96 well-Platte) auf einem Trägersystem, einer Polystyrol-Mikrotiterplatte durch Adsorption bei 37°C in unterschiedlichen Zeiträumen (1h, 2h, 4h, 16h, 48h) immobilisiert und der Träger anschliessend entweder mit 1% BSA oder 1% BSA, 0.5% Tween 20 in 100 mM PBS für 60 min blockiert. Verschiedene Probensubstanzen mit Bakterien (E.coli spec., Citrobacter spec., in LB-Medium, Milch oder Gülle) wurden damit inkubiert (60 min, 37°), anschliessend wurde die Probe durch Waschen mit PBS entfernt (3-4x mit 100 mM PBS, 0.5%Tween 20, je 250µl für LB-Medium, Milch, für Gülle 4-6x) und die gebundenen Bakterien über die bakterieneigene β-Galaktosidase mit β-Gal-Luminol (Tropix, Applied Biosystems) nach Herstellervorschrift nachgewiesen. Der Test ermöglicht je nach Wahl des Substrates eine Empfindlichkeit von unter 100 Zellen/ml in insgesamt unter 4 Stunden.

### Beispiel 4: Gruppenspezifischer Nachweis über Marker

Dazu wurde p12 (300ng/well in 150 µl 100 mM PBS (4mM KH₂PO₄, 16 mM Na₂PO₄, 115 mM NaCl); 96 well-Platte) auf einem Trägersystem, einer Polystyrol-Mikrotiterplatte durch Adsorption bei 37°C in unterschiedlichen Zeiträumen (1h, 2h, 4h, 16h, 48h) immobilisiert und der Träger anschliessend entweder mit 1% BSA oder 1% BSA, 0.5% Tween 20 in 100 mM PBS für 60 min blockiert. Verschiedene Probensubstanzen mit Bakterien (E.coli spec., Citrobacter spec., in LB-Medium, Milch oder Gülle) wurden damit inkubiert (60 min, 37°), anschliessend wurde die Probe durch Waschen mit PBS entfernt (3-4x mit 100 mM PBS, 0.5%Tween 20, je 250µl für LB-Medium, Milch, für Gülle 4-6x). Anschliessend wurde biotinyliertes Phagenschwanzprotein mit Streptavidin gekoppelter Peroxidase gekoppelt. Dieser Komplex wurde zu den gebundenen Bakterien gegeben und verschieden lang inkubiert (10 min, 20 min, 30 min, 60 min). Nach Waschen, 3-4x mit 100 mM PBS, 0.5% Tween 20, je 250µl wurden die gebundenen Bakterien unter Verwendung des ECL-Kits von Amersham nach Hertellerangaben detektiert. Nachgewiesen wurden auf diesem Weg unter 1000 Zellen pro ml.

## Patentansprüche

1. Verfahren zum gleichzeitigen Nachweis verschiedener Bakterien, umfassend die folgenden Schritte
a) Kopplung von einer oder mehrerer Arten von Bakteriophagenschwanzproteinen an einen Träger,
b) Inkubieren des mit den Bakteriophagenschwanzproteinen gekoppelten Trägers mit einer Probe,
c) gegebenenfalls Entfernen der Probe und der nicht an die Bakteriophagenschwanzproteine gebundenen Bakterien der Probe,
d) In-Kontakt-Bringen der an die Bakteriophagenschwanzproteine gebundenen Bakterien mit die nachzuweisenden Bakterien spezifisch bindenden Bakteriophagen und/oder Bakteriophagenproteinen,
e) Entfernen der nicht an die Bakterien gebundenen Bakteriophagen und/oder Bakteriophagenproteinen,
f) Durchführen der Nachweisreaktion mittels eines an die spezifisch bindenden Bakteriophagen und/oder Bakteriophagenproteine gekoppelten Enzyms oder eines Immunnachweises.

2. Verfahren nach Anspruch 1, wobei die Bakteriophagenschwanzproteine mittels Adsorption, mittels chemischer Bindung oder über weitere Proteine an den Träger gekoppelt sind.

3. Verfahren nach einem der Ansprüche 1 bis 2, wobei die Bakteriophagenschwanzproteine biotinyliert und über Streptavidin oder Streptavidin/Avidin-Varianten gebunden werden.

4. Verfahren nach einem der Ansprüche 1 bis 3, wobei die Bakteriophagenschwanzproteine das T4 p12, T2 p12, K3 p12 oder die kurzen Bakteriophagenschwanzproteine der Phagen Felix 01, P1 oder PB1 oder deren Derivate oder Fragmente sind.

5. Verfahren nach einem der Ansprüche 1 bis 4, wobei die Bakteriophagenschwanzproteine die Aminosäuresequenz gemäß SEQ ID NO:1 oder 3 aufweisen.

6. Verfahren nach einem der Ansprüche 1 bis 5, wobei die Bakteriophagenschwanzproteine Modifikationen aufweisen.

7. Verfahren nach einem der Ansprüche 1 bis 6, wobei die Bakteriophagenschwanzproteine einer einzigen oder mehrerer Phagen-Arten mindestens zwei verschiedene Bakterienarten und/oder -gattungen erkennen.

8. Verfahren nach einem der Ansprüche 1 bis 7, wobei der Träger eine Mikrotiterplatte, ein Teststreifen, Objektträger, Wafer, Filtermaterial oder eine Durchflußzellkammer ist und aus Polystyrol, Polypropylen, Polycarbonat, PMMA, Celluloseacetat, Nitrozellulose, Glas oder Siliziumwafer besteht.

9. Verwendung des Verfahrens nach einem der Ansprüche 1 bis 8 zum Nachweis von Bakterien in der Medizin, Lebensmittelindustrie und -analytik, Tierzucht, Trinkwasser-oder Umweltanalytik.

10. Kit zur Durchführung des Verfahrens nach einem der Ansprüche 1 bis 8, umfassend einen Träger mit immobilisierten Bakteriophagenschwanzproteinen, ferner die nachzuweisenden Bakterien spezifisch bindende Bakteriophagen und/oder Bakteriophagenproteine und weitere Testreagentien zum Nachweis der gebundenen Bakterien.

## Claims

1. Method for the simultaneous detection of different bacteria, comprising the steps
a) coupling one or more species of bacteriophage tale proteins onto a support,
b) incubating said support coupled with said bacteriophage tale proteins with a sample,
c) optionally removing said probe and the bacteria not bound to said bacteriophage tale proteins,
d) contacting of the bacteria bound to said bacteriophage tale proteins with bacteriophages and/or bactereriophage proteins, specifically binding the bacteria to be detected,
e) removing said bacteriophages and/or bacteriophage proteins not bound to the bacteria,
f) performing the detection reaction by means of an enzyme coupled to said specifically binding bacteriophages and/or bacteriophage proteins or by means of an immuno assay.

2. Method according to claim 1, wherein said bacteriophage tale proteins are coupled to said support via adsorption, via chemical binding or via further proteins.

3. Method according to any of claims 1 to 2, wherein said bacteriophage tale proteins are biotinylated and bound via Streptavidin or Streptavidin/Avidin variants.

4. Method according to any of claims 1 to 3, wherein said bacteriophage tale proteins are T4 p12, T2 p12, K3 p12 or the short bacteriophage tale proteins of the phages Felix 01, P1 or PB1 or derivatives or fragments thereof.

5. Method according to any of claims 1 to 4, wherein said bacteriophage tale proteins exhibit the amino acid sequence according to SEQ ID NO:1 or 3.

6. Method according to any of claims 1 to 5, wherein said bacteriophage tale proteins exhibit modifications.

7. Method according to any of claims 1 to 6, wherein said bacteriophage tale proteins of one or more phage species detect at least two different bacterial species and/or geni.

8. Method according to any of claims 1 to 7, wherein said support is a microtiter plate, a test strip, slide, wafer, filter material or a flow through cell chamber, and composed of polystyrene, polypropylene, polycarbonate, PMMA, cellulose acetate, nitrocellulose, glas or silicium wafer.

9. Use of the method according to any of claims 1 to 10 for the detection of bacteria in medicine, food industry and analytics, animal breeding, drinking water analytics or environmental analytics.

10. Kit for performing the method according to any of claims 1 to 11, comprising a support with immobilized bacteriophage tale proteins, furthermore bacteriophages and/or bactereriophage proteins specifically binding the bacteria to be detected, and further assay reagents for detection of the bound bacteria

## Revendications

1. Procédé pour la mise en évidence simultanée de différentes bactéries, comprenant les étapes suivantes
a) Couplage d'une ou plusieurs sortes de protéines de queue de bactériophages à un support,
b) Incubation du support couplé aux protéines de queue de bactériophages avec un échantillon,
c) Le cas échéant retrait de l'échantillon et des bactéries de l'échantillon qui ne sont pas liées aux protéines de queue de bactériophages,
d) Mise en contact des bactéries liées aux protéines de queue des bactériophages avec des bactériophages et/ou des protéines de bactériophages se liant de manière spécifique aux bactéries à mettre en évidence,
e) Retrait des bactériophages et/ou des protéines bactériophages non liés aux bactéries,
f) Réalisation de la réaction de mise en évidence par l'intermédiaire d'une enzyme couplée aux bactériophages et/ou aux protéines bactériophages se liant de manière spécifique ou d'un test de mise en évidence immunitaire.

2. Procédé selon la revendication 1, dans lequel les protéines de queue de bactériophages sont couplées par absorption, par liaison chimique ou par d'autres protéines au support.

3. Procédé selon l'une quelconque des revendications 1 à 2, dans lequel les protéines de queue de bactériophages sont biotynilées, et liées par l'intermédiaire de la streptavidine ou de variantes de la streptavidine/avidine.

4. Procédé selon l'une quelconque des revendications 1 à 3, dans lequel les protéines de queue de bactériophages sont T4 p12, T2 p12, K3 p12 ou les protéines courtes de queue de bactériophages du phage Félix 01, P1 ou PB1 ou des dérivés ou des fragments de celles-ci.

5. Procédé selon l'une quelconque des revendications 1 à 4, dans lequel les protéines de queue de bactériophages présentent des séquences d'acides aminés selon la SEQ ID NO : 1 ou 3.

6. Procédé selon l'une quelconque des revendications 1 à 5, dans lequel les protéines de queue de bactériophages présentent des modifications.

7. Procédé selon l'une quelconque des revendications 1 à 6, dans lequel les protéines de queue de bactériophages d'une ou plusieurs espèces de phages reconnaissent au moins deux espèces et/ou genres de bactéries différents.

8. Procédé selon l'une quelconque des revendications 1 à 7, dans lequel le support -est une plaque de microtitration, une bandelette test, une lame porte-objet, une fine lamelle de silicium, du matériau de filtre, ou une chambre cellulaire à circulation et est composé de polystyrène, de polypropylène, de polycarbonate, de PMMA, d'acétate de cellulose, de nitrocellulose, de verre ou d'une fine lame de silicium.

9. Utilisation du procédé selon l'une quelconque des revendications 1 à 8 pour la mise en évidence de bactéries en médecine, en industrie alimentaire et en analyse alimentaire, dans l'élevage, dans l'analyse de l'eau potable et de l'environnement.

10. Coffret pour la réalisation du procédé selon l'une quelconque des revendications 1 à 8, comprenant un support avec des protéines de queue de bactériophages immobilisées, ainsi que les bactériophages et/ou les protéines de bactériophages se liant de manière spécifique aux bactéries à mettre en évidence et d'autres réactifs de tests pour la mise en évidence des bactéries liées.
